# EUROPÄISCHE PATENTANMELDUNG

(11) **EP 3 492 165 A1**
(43) Veröffentlichungstag der Anmeldung: **05.06.2019**
(21) Anmeldenummer: 17204413.3
(22) Anmeldetag: 29.11.2017
(51) Int. Cl.: B01J 8/22, B01J 8/10, B01J 8/02, C07C 29/152, B01J 8/00

(54) **REAKTORKOMPLEX ZUR UMSETZUNG GLEICHGEWICHTSLIMITIERTER REAKTIONEN UND VERFAHREN HIERZU**

(71) Anmelder: Siemens Aktiengesellschaft, 80333 München (DE)
(72) Erfinder: Stark, Katharina, 96215 Lichtenfels (DE); Baldauf, Manfred, 91056 Erlangen (DE); Tremel, Alexander, 91096 Möhrendorf (DE)

(57) **Zusammenfassung**

Die Erfindung betrifft einen Reaktorkomplex zur Umsetzung gleichgewichtslimitierter Reaktionen umfassend eine Eduktzuführvorrichtung (4), einen Reaktionsraum (6) mit einer Katalyseeinheit (8) und eine Fördervorrichtung (10) für ein flüssiges Sorptionsmittel (12), wobei die Fördervorrichtung eine Pumpvorrichtung (14) und eine Entladevorrichtung (16) für ein Reaktionsprodukt (18) aufweist. Die Erfindung zeichnet sich dadurch aus, dass eine Messvorrichtung (20) zur Messung eines Beladungszustandes des Sorptionsmittels (12) mit dem Reaktionsprodukt (18) vorgesehen ist und dass eine Regel- oder Steuervorrichtung (22) vorgesehen ist, die mit der Messvorrichtung (20) in Verbindung steht und die wiederum zur Regelung oder Steuerung der Pumpvorrichtung (14) dient.

## Beschreibung

Die Erfindung betrifft einen Reaktorkomplex zur Umsetzung gleichgewichtslimitierter Reaktionen nach dem Oberbegriff des Patentanspruchs 1 sowie ein Verfahren zur Umsetzung gleichgewichtslimitierter Reaktionen nach Anspruch 7.

Durch Zunahme des Anteils regenerativ erzeugter Energien im Stromnetz tritt vermehrt, insbesondere wetterabhängig, ein Energieüberschuss in Form von elektrischer Energie auf. Eine Möglichkeit, diesen Energieüberschuss zu kompensieren bzw. anderweitig zu nutzen, besteht neben der Speicherung an sich darin, diesen Energieüberschuss in die Herstellung von Wertstoffen, insbesondere chemischen Wertstoffen zu binden. Hierbei hat sich z. B. die Methanolsynthese aus Kohlendioxid, das ohnehin bei der Erzeugung von elektrischer Energie aus fossilen Brennstoffen anfällt und aus Wasserstoff bewährt. Dies erfolgt im Grundsatz nach der chemischen Gleichung

CO₂ + 3 H₂ → CH₃OH + H₂O + ΔE Gleichung 1

Ein Aspekt einer Synthese von Methanol aus Kohlendioxid und Wasserstoff sind niedrige Gleichgewichtsumsätze, d. h. das chemische Gleichgewicht nach der Gleichung 1 liegt im Wesentlichen auf der linken Seite. Daher muss ein großer Teil der gasförmigen Edukte im Kreis geführt werden, was wiederum zu Druckverlusten im Reaktor und in Rohrleitungen führt, und letztendlich durch Zusatz von weiterer elektrischer Energie ausgeglichen werden muss.

In der DE 10 2015 202 681 A1 wird ein Reaktor und ein Verfahren beschrieben, in dem die genannte Methanolsynthese unter Zuhilfenahme von Katalysatormaterialien und von Sorptionsmittel, die die Reaktionsprodukte aus der Gasphase aufnehmen und somit das sich eingestellte chemische Gleichgewicht gemäß Gleichung 1 in derart verändern, dass es sich erneut durch Umsetzung der Edukte in Produkte entsprechend neu einstellt. Das Sorptionsmittel wird dabei mit dem Reaktionsprodukt beladen, bis es letztendlich durch die absorbierten Reaktionsprodukte gesättigt ist. Die durch das Sorptionsmittel aufgenommenen Reaktionsprodukte, gemäß Gleichung 1 Methanol und Wasser, werden dann im Weiteren in einem Entladeprozess wieder dem Sorptionsmittel entzogen. Das in dieser Weise entladene Sorptionsmittel wird bevorzugt wieder dem Reaktionsprozess zugeführt und in den Reaktor eingebracht.

Bei der Auswahl eines geeigneten Sorptionsmittels ist insbesondere darauf zu achten, dass dieses ein hohes Aufnahmevermögen für die Reaktionsprodukte Methanol und Wasser aufweist. Dabei ist es zweckmäßig, dass das Sorptionsmittel solange wie möglich im Reaktionsraum des Reaktors verweilt, um eine möglichst große Menge der Reaktionsprodukte aufzunehmen. Allerdings darf die Verweilzeit im Reaktor auch nicht zu hoch sein, da mit zunehmender Sättigung der Absorptionsflüssigkeit die treibende Kraft der Absorption abnimmt. Aus diesem Grund muss die Beladung des Sorptionsmittels mit den Produkten so gewählt werden, dass eine effektive Aufnahme von Methanol und H₂O aus dem Gas ermöglicht werden kann.

Die Aufgabe der Erfindung besteht darin, einen Reaktorkomplex zur Umsetzung gleichgewichtslimitierter Reaktionen sowie ein entsprechendes Verfahren bereitzustellen, bei dem gegenüber dem Stand der Technik die Ausbeute an Reaktionsprodukten bei einer gleichgewichtslimitierten Reaktion gesteigert wird.

Die Lösung der Aufgabe besteht in einem Reaktorkomplex zur Umsetzung gleichgewichtslimitierter Reaktionen mit den Merkmalen des Patentanspruchs 1 sowie in einem Verfahren zu Umsetzung gleichgewichtslimitierter Reaktionen nach Patentanspruch 7.

Der erfindungsgemäße Reaktorkomplex zur Umsetzung gleichgewichtslimitierter Reaktionen nach Anspruch 1 umfasst eine Eduktzuführvorrichtung, einen Reaktionsraum mit einer Katalysatoreinheit und eine Fördervorrichtung für ein flüssiges Sorptionsmittel. Die Fördervorrichtung weist dabei eine Pumpvorrichtung und eine Entladevorrichtung für ein Reaktionsprodukt auf. Die Erfindung zeichnet sich dadurch aus, dass eine Messvorrichtung zur Messung eines Beladungszustandes des Sorptionsmittels mit dem Reaktionsprodukt vorgesehen ist. Ferner ist eine Regel- oder Steuervorrichtung vorgesehen, die wiederum mit der Messvorrichtung in Verbindung steht. Die Regel- oder Steuervorrichtung wiederum steht mit der Pumpvorrichtung in Verbindung und dient zur Regelung und Steuerung dieser Pumpvorrichtung.

Unter dem Begriff in Verbindung stehen wird hierbei verstanden, dass eine geeignete Verbindung zur Übertragung eines Mess-, Regelungs- oder Steuersignals besteht. Diese geeignete Verbindung kann in Form von verschiedenen Übertragungsmitteln, wie Kabel, Glasfaser oder Funk, wie WLAN oder Bluetooth bestehen.

Durch die Messung des Beladungszustandes des Sorptionsmittels mit dem Reaktionsprodukt und durch die Verarbeitung eines hierbei auftretenden Messsignals mit der Steuer- oder Regelungsvorrichtung ist ein gezielter Eingriff auf die Fördervorrichtung, insbesondere auf die Pumpvorrichtung möglich, sodass ein dynamischer Betrieb des Reaktors und der Sorptionsmittelumwälzung möglich ist, bei dem jeweils der Beladungszustand des Sorptionsmittels so hoch ist, dass es sich um ein optimales Aufnahmevermögen, handelt und dabei die Ausbeute an Reaktionsprodukten gegenüber dem Verfahren und dem Reaktor nach dem Stand der Technik erhöht wird. Das optimale Aufnahmevermögen liegt meist in einem Bereich zwischen 70 % und 90 % des maximalen Aufnahmevermögens.

Grundsätzlich kommt bei der Auswahl des Messverfahrens für die Messvorrichtung eine Vielzahl von Parametern in Frage. Somit kann die Messvorrichtung in der Form ausgestaltet sein, dass beispielsweise die Viskosität oder die Dichte des Sorptionsmittels gemessen wird. Ferner kann auch die Schallausbreitung oder die Wärmekapazität ein hilfreiches Maß für den Beladungszustand des Sorptionsmittels sein. Aus all den genannten Werten kann ein entsprechendes Messsignal erzeugt werden, dass anhand einer Kalibrierungskurve den Beladungszustand des Sorptionsmittels mit dem Reaktionsprodukt wiedergibt. Als besonders vorteilhaft hat sich jedoch herausgestellt, dass elektrische Messgrößen, die mithilfe von Elektroden bestimmt werden können, besonders gut geeignet sind, den Beladungszustand zu ermitteln. Derartige sind insbesondere die elektrische Leitfähigkeit, das Elektrodenpotential oder die Permittivität. Zur Aufnahme dieser nicht abschließend aufgeführten elektrischen Kenngrößen ist es zweckmäßig und besonders vorteilhaft, zwei Elektroden als Bestandteil der Messvorrichtung einzusetzen. Dabei ist es wiederum besonders zweckmäßig, dass mindestens eine Elektrode im direkten Kontakt mit dem flüssigen Sorptionsmittel steht. Eine andere, zweite Elektrode kann gegebenenfalls auch außerhalb des flüssigen Sorptionsmittels angeordnet sein.

Der Reaktorkomplex weist einen Reaktionsraum und einen Sammelraum zur Aufnahme und Sammlung des Sorptionsmittels auf. Reaktionsraum und Sammelraum befinden sich dabei bevorzugt in einem Gehäuse, das auch als Reaktor bezeichnet werden kann. Der Sammelraum befindet sich dabei bevorzugt in einem unteren Bereich des Reaktors, sodass das Sorptionsmittel allein durch die Schwerkraft sich dort anreichert. Dabei ist es zweckmäßig, wenn zumindest die erste Elektrode in den Sammelraum so angeordnet ist, dass sie bevorzugt unterhalb eines stetigen Spiegels des flüssigen Sorptionsmittels liegt und somit mit diesen stets in Kontakt steht. Auch die zweite Elektrode kann dabei unterhalb des Sorptionsmittelspiegels angeordnet sein. Ein elektrisches Signal zwischen den beiden Elektroden, die wiederum Teil der Messvorrichtung sind, kann entsprechend ausgewertet werden und an die Regelungs- oder Steuerungsvorrichtung weitergeleitet werden.

Unter dem Begriff Steuerungsvorrichtung wird eine Vorrichtung verstanden, die ein Signal an eine Prozesskomponente weitergibt, worauf diese Prozesskomponente, in diesem Fall die Pumpvorrichtung gezielt gesteuert wird. Eine Rückmeldung über den Prozess von dem Prozessgerät an die Steuerungsvorrichtung erfolgt dabei nicht. Dies ist allerdings bei der Regelungsvorrichtung der Fall, indem ein Regelungssignal an das Prozesselement gegeben wird und abhängig von einem voreingestellten Istwert eine Rückmeldung des Prozesselementes an die Regelungsvorrichtung und eine Anpassung des Regelungs- bzw. Steuersignales erfolgt. Je nach Gestaltung des Prozesses kann somit jeweils eine Regelungs- oder Steuerungsvorrichtung zweckmäßig sein. Unter dem Begriff Pumpenvorrichtung wird grundsätzlich eine Vorrichtung verstanden, die dazu geeignet ist, ein Fluid in einer Leitung bzw. in einem Raum voranzutreiben. Dies ist üblicherweise eine Pumpe, es können jedoch auch andere Bauteile beispielsweise eine Membran oder lediglich der Einsatz einer schwerkraftgetriebenen Vorrichtung zweckmäßig sein. Im Falle einer schwerkraftgetriebenen Pumpvorrichtung zum Vorantreiben des Sorptionsmittels könnte beispielsweise mit Hilfe des Regelungs- oder Steuersignals ein Querschnitt eines Fallrohres eingestellt werden, um so den Sorptionsmittelfluss zu regulieren.

Die Fördervorrichtung weist, wie bereits erwähnt, ein Leitungssystem auf, das außerhalb des Reaktorraums angeordnet ist und das zweckmäßigerweise zu einer Entladungsvorrichtung führt. Grundsätzlich ist es auch zweckmäßig, dass die Messvorrichtung und zumindest die erste Elektrode im oder an dem Leitungssystem angeordnet ist und dort in Kontakt mit dem Sorptionsmittel steht. Ferner ist es zweckmäßig, dass eine zusätzliche Bypassleitung vorgesehen ist, die zu der Fördervorrichtung, zu der auch der Sammelraum gehört, angeordnet ist. Durch diese Bypassleitung wird ein Teil des Sorptionsmittels in Form eines Bypassstromes geleitet, sodass auch hierdurch in situ der Beladungszustand des Sorptionsmittels durch die Messvorrichtung in oder an der Bypassleitung gemessen werden kann. Hierbei ist es auch zweckmäßig, dass die erste Elektrode in Kontakt mit dem flüssigen Sorptionsmittel steht.

Ein weiterer Bestandteil der Erfindung ist ein Verfahren zum Betreiben eines Reaktorkomplexes zur Umsetzung gleichgewichtslimitierter Reaktionen. Hierbei wird mindestens ein Edukt durch eine Eduktzuführvorrichtung in einen Reaktionsraum und in eine darin angeordnete Katalyseeinheit geleitet. Dort wird das Edukt bis zum Erreichen eines Gleichgewichtszustandes zu einem Reaktionsprodukt umgesetzt, wobei das Reaktionsprodukt im Weiteren von einem flüssigen Sorptionsmittel absorbiert wird. Das mit dem Reaktionsprodukt beladene Sorptionsmittel wird mithilfe einer Fördervorrichtung, umfassend eine Pumpvorrichtung und eine Entladevorrichtung aus dem Reaktionsraum gefördert und vom Reaktionsprodukt entladen. Die Erfindung zeichnet sich dadurch aus, dass eine Messvorrichtung vorgesehen ist, durch die ein Beladezustand des Sorptionsmittels gemessen wird und ein Messsignal an eine Regel- oder Steuervorrichtung gesendet wird. Durch die Regel- oder Steuervorrichtung wird eine Pumpvorrichtung geregelt oder gesteuert, und somit der Volumenstrom des beladenen Sorptionsmittels entsprechend eingestellt.

Das erfindungsgemäße Verfahren weist grundsätzlich dieselben vorteilhaften Eigenschaften auf, die bereits bezüglich des Reaktors erörtert sind. Hierbei handelt es sich insbesondere um die Steigerung des Wirkungsgrades des Reaktors, da durch die gezielte und stetige Entladung des Sorptionsmittels das Reaktionsgleichgewicht vorteilhafterweise verschoben wird.

Es hat sich als zweckmäßig herausgestellt, dass eines der Edukte Kohlendioxid ist, ein weiteres Edukt ist dabei Wasserstoff. In einer vorteilhaften Umsetzung entsteht hieraus Methanol. Bei dem Sorptionsmittel handelt es sich bevorzugt um eine ionische Flüssigkeit, die sehr gute Aufnahmeeigenschaften für Methanol, Wasser und auch andere Produkte aufweisen.

Es hat sich herausgestellt, dass eine bevorzugte Reaktionstemperatur im Reaktionsraum bei 200° C bzw. 300° C liegt, dabei ist ein Reaktionsdruck im Reaktionsraum zwischen 5 MPa und 10 MPa, Drücke bis 250 MPA sind auch möglichebenfalls für eine wirtschaftliche Ausbeute an Reaktionsprodukten vorteilhaft.

Weitere Ausgestaltungsformen der Erfindung und weitere Merkmale werden anhand der folgenden Figuren näher beschrieben. Dabei handelt es sich um rein exemplarische Darstellungen, die keine Einschränkung des Schutzbereichs darstellen. Merkmale mit derselben Bezeichnung aber in unterschiedlichen Ausgestaltungsformen erhalten dabei dasselbe Bezugszeichen. Dabei zeigen:
Figur 1 einen Reaktor mit einem Reaktionsraum und einer Katalyseeinheit sowie einen Sammelraum für Sorptionsmittel und eine Fördervorrichtung für das Sorptionsmittel mit einer Messvorrichtung, die in Sammelbehälter angeordnet ist,
Figur 2 einen Reaktorkomplex gemäß Figur 1, wobei die Messvorrichtung in einen Leitungssystem der Fördervorrichtung außerhalb des Sammelraums angeordnet ist,
Figur 3 einen Reaktorkomplex gemäß Figur 1 mit einer Bypassleitung und einer darin angeordneten Messvorrichtung und
Figur 4 einen Reaktorkomplex gemäß Figur 1 mit einer alternativen Messvorrichtung.

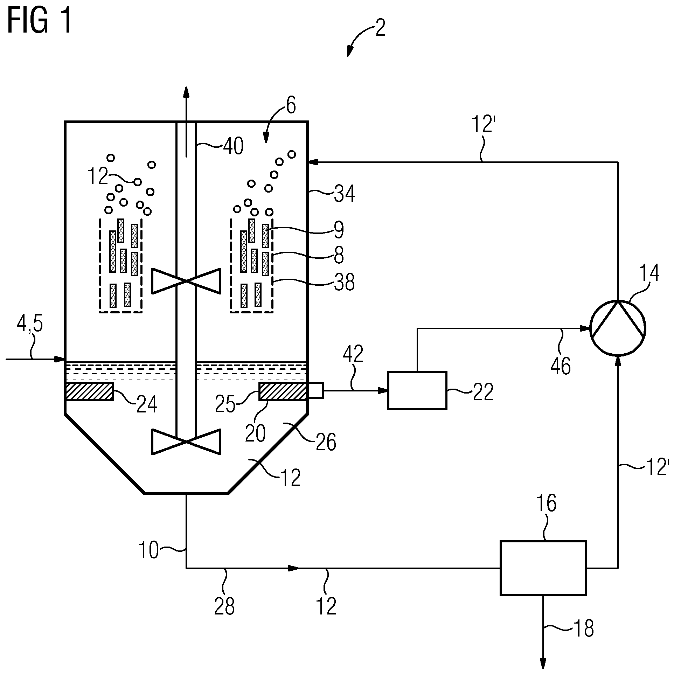

In Figur 1 ist ein Reaktorkomplex 2 dargestellt, der dazu geeignet ist, gleichgewichtslimitierte Reaktionen mit einem hohen Wirkungsgrad zu betreiben. Dieser Reaktorkomplex 2 weist ein Reaktorgehäuse 34 auf, in dem ein Reaktionsraum 6 sowie ein Sammelraum 26 für ein Sorptionsmittel 12 vorgesehen sind. Ferner weist das Reaktorgehäuse 34 eine Eduktzuführvorrichtung 4 auf, durch das ein Edukt 5 (in der Regel CO2 und H2) in den Reaktionsraum 6 gelangt. Im Reaktionsraum 6 ist eine Katalyseeinheit 8 angeordnet, in der ein Katalysatormaterial 9 in der Reaktionsform entsprechenden Art und Weise angeordnet ist. Dies kann beispielsweise eine Schüttung von Katalysatormaterial 9 sein.

In einem oberen Bereich des Reaktionsraumes 6 wird ein Sorptionsmittel 12', das beispielsweise in Form einer ionischen Flüssigkeit ausgestaltet sein kann, eingebracht. Die Edukte 5 strömen, da sie bei den Reaktionsbedingungen von etwa 100° C bis 200° C in einem Druckbereich von 5 MPa bis 10 MPa gasförmig vorliegen, durch die Katalyseeinheit 8, worauf eine chemische Reaktion, insbesondere die Reaktion von Kohlendioxid und Wasserstoff zu Methanol und Wasser gemäß Gleichung 1 stattfindet. Diese Reaktion hat ein Gleichgewicht auf der linken Seite, also auf der Seite der Edukte 5, sodass ohne weitere Maßnahmen lediglich etwa 20 % der Edukte zu Reaktionsprodukten 18 umgewandelt werden. Durch den Kontakt der Reaktionsprodukte 18 mit dem Sorptionsmittel 12' werden diese Reaktionsprodukte 18 von dem Sorptionsmittel 12' aufgenommen, man spricht dabei davon, dass das Sorptionsmittel 12' nun mit den Reaktionsprodukten 18 beladen ist. Dieses beladene Sorptionsmittel wird im Weiteren mit dem Bezugszeichen 12 versehen. Das Sorptionsmittel 12' und 12 liegt bei den Reaktionsbedingungen in flüssiger Form vor und sinkt im Reaktorgehäuse 34 der Schwerkraft folgend in einen Sammelraum 26 ab, der im unteren Bereich des Reaktorgehäuse 34 angeordnet ist. Die Bereitstellung eines Sammelraums 26 für das Sorptionsmittel 12 ist zweckmäßig, da das Sorptionsmittel 12 hierbei durchmischt werden kann, wobei sich eine gleichmäßige Verteilung des beladenen Reaktionsproduktes 18 einstellt. Außerdem ist eine am Boden des Reaktors vorliegende Flüssigkeit als sogenannte Gassperre zweckmäßig. Gewöhnlich ist am Boden des Reaktors ein nicht dargestelltes Druckhalteventil angeordnet. Wenn das Ventil am Boden des Reaktors öffnet, kann so verhindert werden, dass Gas nach unten austritt. Deswegen sollte stets ein Flüssigkeitspegel am Boden des Reaktors vorliegen. Für die Durchmischung des Sorptionsmittels 12 im Sammelraum 26 sowie bevorzugt auch im Bereich der Katalyseeinheit 8 ist eine Rührvorrichtung 40, bevorzugt mit zwei Rührblättern ausgestaltet, vorgesehen.

Im Weiteren weist der Reaktorkomplex neben dem Reaktorgehäuse eine Fördervorrichtung 10 zur Förderung des Sorptionsmittels 12 auf. Zu der Fördervorrichtung 10 wird dabei der Sammelraum 26, der sich innerhalb des Reaktorgehäuses 34 befindet, gezählt, da er ein Reservoir an Sorptionsmittel 12 bildet. Ferner umfasst die Fördervorrichtung 10 ein Leitungssystem 28, das bevorzugt an das Reaktorgehäuse 34 angeflanscht ist, und durch das das Sorptionsmittel 12 im beladenen Zustand in eine Entladevorrichtung 16 geleitet wird. Die Entladevorrichtung 16 wird hier nicht näher beschrieben, sie dient dazu, durch Einstellung geeigneter thermodynamischer Bedingungen, wie Druck und Temperatur, das Reaktionsprodukt, das in diesem Fall aus Methanol und Wasser besteht, vom Sorptionsmittel zu trennen. Im Weiteren wird das entladene Sorptionsmittel 12' wieder wie beschrieben im oberen Teil des Reaktorgehäuses 34 eingeleitet, wobei auf dem Weg von der Entladevorrichtung 16 bis zum Wiedereintritt in das Reaktorgehäuse 34 eine Pumpvorrichtung 14 angeordnet ist. Die Pumpvorrichtung 14 ist dabei in den Figuren 1 bis 4 an dieser Stelle lediglich exemplarisch dargestellt, sie kann je nach genauem Aufbau des Reaktorkomplexes 2 an unterschiedlichen Stellen der Fördervorrichtung 10 angeordnet sein. Bei der Pumpvorrichtung muss es sich auch nicht zwangsweise um eine Pumpe an sich und im weiteren Sinne handeln, es können alle Maßnahmen, die dazu geeignet sind, das flüssige Sorptionsmittel in Bewegung zu setzen, als Pumpvorrichtung 14 bezeichnet werden.

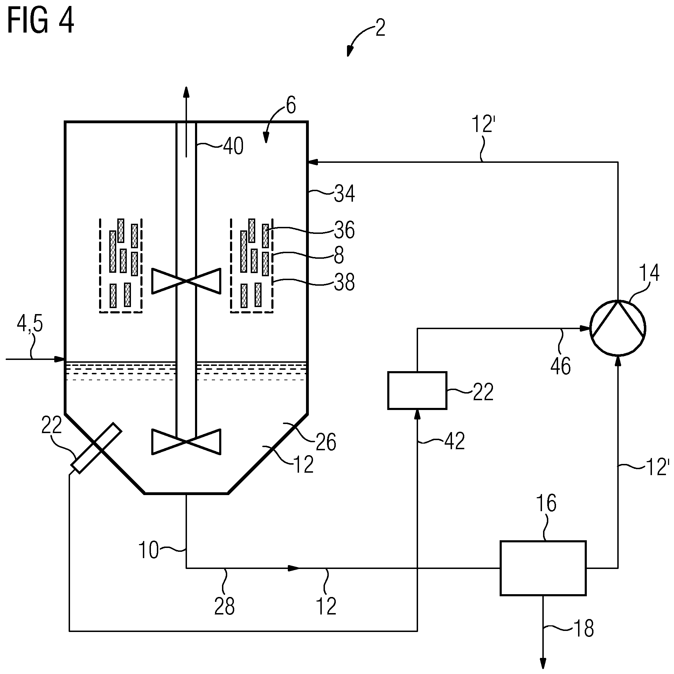

Der Reaktorkomplex 2 gemäß Figur 1 weist im Weiteren eine Messvorrichtung 20 auf, die grundsätzlich eine Vielzahl von Stoffeigenschaften des beladenen Sorptionsmittels 12 ermitteln kann. Typische und auch zweckmäßige Bestimmungsgrößen des Sorptionsmittels sind dabei dessen Viskosität, dessen Dichte, dessen Schallausbreitungsfähigkeit sowie deren Wärmekapazität. Eine allgemeine Messvorrichtung für derartige Messgrößen ist exemplarisch in Figur 4 dargestellt. Besonders zweckmäßig ist es jedoch, wenn elektrische Messgrößen, die mithilfe von Elektroden induziert und aufgenommen werden können, Bestandteil der Messvorrichtung 20 sind. Derartige Messgrößen sind insbesondere aber nicht abschließend die elektrische Leitfähigkeit des Sorptionsmittels, das Elektrodenpotential oder die relative Permittivität, die wiederum auch frequenzabhängig gemessen werden kann. Hierzu sind gemäß Figur 1 zwei Elektroden 24 und 25 im Sammelraum 26 angeordnet. Mindestens eine Elektrode, zumindest die erste Elektrode 24 ist so im Sammelraum 26 angeordnet, dass sie in einem Betriebszustand stets unterhalb eines, hier mit einer gestrichelten Linie eingezeichneten Flüssigkeitsspiegels des flüssigen Sorptionsmittels 12 angeordnet ist. Die Elektroden 24 und 25 sind gemäß Figur 1 lediglich sehr schematisch dargestellt und bilden einen Teil einer nicht weiter dargestellten Messvorrichtung 20. Die Messvorrichtung 20 ist im Weiteren in derart ausgestaltet, dass sie ein Messsignal erzeugt, das wiederum über eine Leitung 42 zu einer Regel- oder Steuervorrichtung 22 weitergibt. Dies kann beispielsweise über Funk, wie Bluetooth oder WLAN, aber auch über eine feste Leitung, die auch optische Fasern einschließt, erfolgen.

Durch die Messung von einer der genannten Stoffeigenschaften des flüssigen Sorptionsmittels 12 und der Reaktionsbedingungen, bei einer Beladung von 0 % bis zu einer Beladung von 100 %, kann eine Kalibrierkurve erstellt werden. Über diese Kalibrierkurve kann der Beladungsgrad des Sorptionsmittels 12 mit den Reaktionsprodukten im Reaktor bestimmt werden. Dieser Beladungsgrad wird im vorliegenden Beispiel gemäß Figur 1 über die Elektroden 24 und 25 in Form der elektrischen Leitfähigkeit gemessen. Dabei wird bei der vorgestellten Messmethode ausgenutzt, dass durch das Beladen des Sorptionsmittels mit Methanol und Wasser dessen elektrischen Widerstand in der flüssigen Phase verändert wird. Die Messung der elektrischen Leitfähigkeit ist besonders sinnvoll, wenn als Sorptionsmittel 12 ein Salz, z. B. eine ionische Flüssigkeit eingesetzt wird. Salze haben aufgrund ihres ionischen Charakters eine vergleichsweise hohe elektrische Leitfähigkeit, die sich durch die Einlösung der ungeladenen Moleküle des Methanols und des Wassers erniedrigt. Durch einen analogen Aufbau gemäß Figur 1 kann auch die Permittivität bzw. die elektrische Leitfähigkeit ermittelt werden. Dabei können Alkohol und Wasser aufgrund ihrer vergleichsweisen hohen relativen Permittivität (relative Permittivität *εᵣ* von Methanol ist 33,8 F x m⁻¹ bei 25° C, *εᵣ* von Wasser ist 81,1 F x m⁻¹ bei 18° C) die relative Permittivität des Sorptionsmittels deutlich verändern.

Die mit den beschriebenen Methoden und auf den genannten physikalischen Größen beruhende Kalibrierkurve ist in der Steuervorrichtung 22, die auch als Regelvorrichtung 22 ausgestaltet sein kann, hinterlegt. Aufgrund des Messsignales wird der Beladungszustand des Sorptionsmittels 12 anhand der Kalibrierkurve, die hier nicht dargestellt ist, ermittelt und ein entsprechender Steuerwert an die Pumpvorrichtung 14 weitergeleitet. Durch die Intensität, mit der die Pumpvorrichtung 14 das Sorptionsmittel 12 durch das Leitungssystem 28 pumpt, kann eingestellt werden, wie viel Sorptionsmittel aus dem Reaktionsraum 6 und dem Sammelraum 12 entfernt wird, und wie viel Sorptionsmittel 12', das unbeladen ist, wieder in den Reaktionsraum 6 eingeführt wird. Auf diese Weise kann je nach Reaktionszustand stets Sorptionsmittel 12' mit einem noch optimalen Aufnahmevermögen für Reaktionsprodukte 18 bereitgestellt werden. Verläuft der Kreislauf angetrieben durch die Pumpvorrichtung 14 zu schnell, würde nahezu unbeladenes Sorptionsmittel 12 aus dem Sammelraum 26 abgezogen werden, dass noch deutlich an Reaktionsprodukten aufnehmen könnte. Hierbei würden höhere Energiekosten für die Umwälzung, also für die Pumpvorrichtung 14 entstehen. Weiterhin wäre der Entladeprozess in der Entladevorrichtung 16 ebenfalls unwirtschaftlicher. Ist jedoch zu viel hochbeladenes Sorptionsmittel 12 im Reaktorgehäuse 34 vorhanden, werden zu wenige Reaktionsprodukte im Bereich der Katalyseeinheit 8 vom Sorptionsmittel 12 bzw. 12' aufgenommen, wodurch die Reaktion verlangsamt wird. Auch dies ist negativ für die Wirtschaftlichkeit des ablaufenden Prozesses.

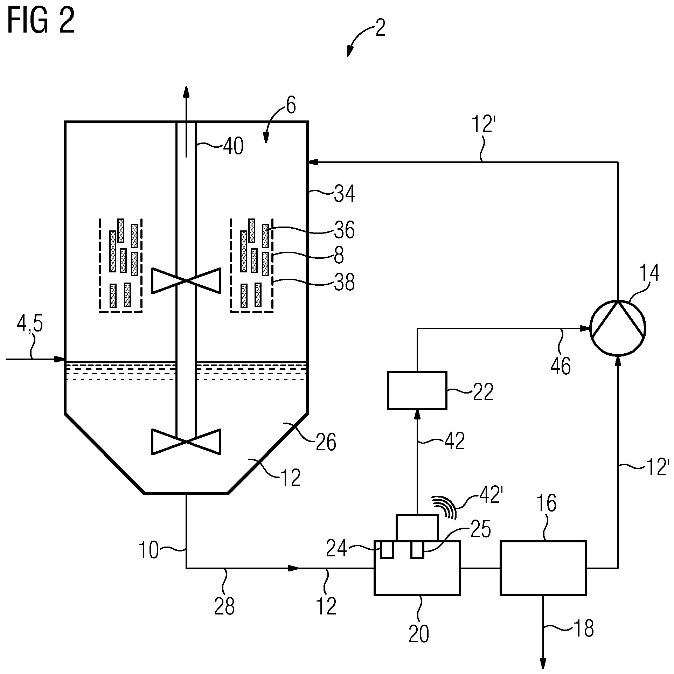

Der in Figur 1 dargestellte Aufbau mit der Messvorrichtung 20 bzw. den Elektroden 24 und 25 im Inneren des Reaktorgehäuses 34, dort im Sammelraum 26 angeordnet, wird als eine in situ Messung bezeichnet. Grundsätzlich ist auch eine ex situ Messung zweckmäßig, hierbei ist die Messvorrichtung 20 außerhalb des Reaktorgehäuses 34 im Leitungssystem 28 angeordnet. Dieser Aufbau ist in Figur 2 abgebildet. Hierbei ist die Messvorrichtung 20 vor der Entladevorrichtung 16 in die Rohrleitung 28 eingekoppelt. Die beiden Elektroden 24 und 25 sind hier ebenfalls im direkten Kontakt mit dem flüssigen Sorptionsmittel 12. Es ist jedoch auch zweckmäßig, wenn lediglich eine Elektrode 24 im Kontakt mit dem flüssigen Sorptionsmittel 12 ist, die zweite Elektrode 25 kann auch außerhalb des Flüssigkeitsstromes angeordnet sein. Auch hier ist zwischen der Messvorrichtung 20 und der Steuerungsvorrichtung 22 eine Verbindung 42 eingezeichnet, die in Form von Kabeln oder auch in Form von Funkwellen darstellbar ist, die mit dem Bezugszeichen 42' versehen sind. Grundsätzlich können die Messvorrichtung 20 und die Steuervorrichtung 22 auch in einem Bauteil bzw. in einem hier nicht dargestellten Gehäuse integriert sein.

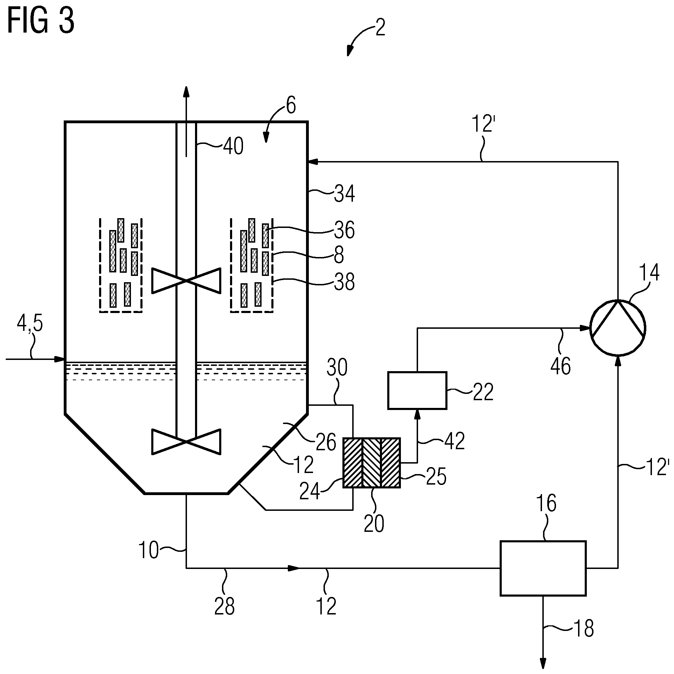

Analog der Figur 3 kann für die elektrochemische Messung des Elektrodenpotentials auch ein brennstoffzellenähnlicher Aufbau verwendet werden. Dazu wird eine als Messschleife dienende Bypassleitung 30 in einen hier nicht dargestellten Anodenraum einer DMFC-Brennstoffzelle geleitet. Dieser Raum wird von der Gegenelektrode (die Elektroden sind an dieser Stelle mit 24 und 25 bezeichnet) durch ein Diaphragma oder eine Membran getrennt. Die Zellspannung bei vorgegebener Stromdichte hängt von der Methanolkonzentration ab. Auch ein potentiostatischer Betrieb ist dabei möglich. Im einfachsten Fall wird stromlos lediglich die Ruhespannung der Elektrodenanordnung 24, 25 gemessen. Grundsätzlich kann es auch zweckmäßig sein, eine Redoxelektrode als Messvorrichtung 20 zu verwenden, um das Redoxpotential des Sorptionsmittels 12 zu messen.

## Patentansprüche

1. Reaktorkomplex zur Umsetzung gleichgewichtslimitierter Reaktionen umfassend eine Eduktzuführvorrichtung (4), einen Reaktionsraum (6) mit einer Katalyseeinheit (8) und eine Fördervorrichtung (10) für ein flüssiges Sorptionsmittel (12), wobei die Fördervorrichtung eine Pumpvorrichtung (14) und eine Entladevorrichtung (16) für ein Reaktionsprodukt (18) aufweist, **dadurch gekennzeichnet, dass** eine Messvorrichtung (20) zur Messung eines Beladungszustandes des Sorptionsmittels (12) mit dem Reaktionsprodukt (18) vorgesehen ist und dass eine Regel- oder Steuervorrichtung (22) vorgesehen ist, die mit der Messvorrichtung (20) in Verbindung steht und die wiederum zur Regelung oder Steuerung der Pumpvorrichtung (14) dient.

2. Reaktorkomplex nach Anspruch 1, **dadurch gekennzeichnet, dass** die Messvorrichtung (20) mindestens zwei Elektroden (24), (25) aufweist, wobei mindestens eine erste Elektrode (24) so angeordnet ist, dass sie in einem betriebsfähigen Zustand des Reaktorkomplexes (2) im direkten Kontakt mit dem flüssigen Sorptionsmittel (12) steht.

3. Reaktorkomplex nach Anspruch 1 oder 2, **dadurch gekennzeichnet, dass** die Messvorrichtung (20) zur Messung der elektrischen Leitfähigkeit, der Permittivität und/oder eines elektrischen Potentials geeignet ist.

4. Reaktorkomplex nach Anspruch 2 oder 3, **dadurch gekennzeichnet, dass** der Reaktionsraum (6) mit einem Sammelraum (26) für das Sorptionsmittel (12) in Verbindung steht und die Elektroden (24, 25) im Sammelraum angeordnet sind.

5. Reaktorkomplex nach Anspruch 2 oder 3, **dadurch gekennzeichnet, dass** die Fördervorrichtung (10) ein Leitungssystem (28) außerhalb des Reaktionsraums (6) und des Sammelraums (26) aufweist und die erste Elektrode (24) in dem Leitungssystem (28) angeordnet ist.

6. Reaktorkomplex nach Anspruch 2 oder 3, **dadurch gekennzeichnet, dass** bezüglich der Fördervorrichtung (10) eine Bypassleitung (30) zur Beförderung eines Bypassstroms des Sorptionsmittels (12) vorgesehen ist und die erste Elektrode (24) der Messvorrichtung (20) in oder an der Bypassleitung (30) angeordnet ist.

7. Verfahren zum Betreiben eines Reaktorkomplexes (2) zur Umsetzung gleichgewichtslimitierter Reaktionen, wobei mindestens ein Edukt (5) durch eine Eduktzuführvorrichtung (4) in einen Reaktionsraum (6) und in eine darin angeordnete Katalyseeinheit (8) geleitet werden und dort bis zum Erreichen eines Gleichgewichtzustandes zu einem Reaktionsprodukt (18) umgesetzt werden, wobei das Reaktionsprodukt (18) im Weiteren von einem flüssigen Sorptionsmittel (12) absorbiert wird und das mit dem Reaktionsprodukt (18) beladene Sorptionsmittel (12) mit Hilfe einer Fördervorrichtung (10), umfassend eine Pumpvorrichtung (14) und eine Entladevorrichtung (16), aus dem Reaktionsraum (6) gefördert wird und vom Reaktionsprodukt (18) entladen wird, **dadurch gekennzeichnet, dass** eine Messvorrichtung (20) vorgesehen ist, durch die ein Beladungszustand des Sorptionsmittels (12) gemessen wird und ein Messsignal an eine Regel- oder Steuervorrichtung (22) gesendet wird und durch die Regel- oder Steuervorrichtung (22) die Pumpvorrichtung (14) zur Einstellung eines Volumenstroms des beladenen Sorptionsmittel (12) geregelt oder gesteuert wird.

8. Verfahren nach Anspruch 7, **dadurch gekennzeichnet, dass** als Edukt (7) Kohlendioxid eingeführt wird.

9. Verfahren nach Anspruch 8, **dadurch gekennzeichnet, dass** als Edukt (7) Kohlendioxid und Wasserstoff eingeführt wird.

10. Verfahren nach Anspruch 7 oder 8, **dadurch gekennzeichnet, dass** als Reaktionsprodukt (18) Methanol erzeugt wird.

11. Verfahren nach einem der Ansprüche 7 bis 10, **dadurch gekennzeichnet, dass** als Sorptionsmittel (12) eine ionische Flüssigkeit verwendet wird.

12. Verfahren nach einem der Ansprüche 7 bis 11, **dadurch gekennzeichnet, dass** die Reaktion in einem Temperaturbereich zwischen 200° C und 300° C betrieben wird.

13. Verfahren nach einem der Ansprüche 7 bis 12, **dadurch gekennzeichnet, dass** die Reaktion bei einem Druck zwischen 5 MPa und 25 MPa liegt, bevorzugt zwischen 5 MPa und 10 MPa.
